# EUROPEAN PATENT SPECIFICATION

(11) **EP 0 681 654 B1**
(45) Date of publication and mention of the grant of the patent: **08.09.1999**
(21) Application number: 92922797.3
(22) Date of filing: 19.10.1992
(51) Int. Cl.: F04B 17/00, A61M 1/10, F04D 13/06, F04D 1/04, H02K 5/16, H02K 7/09

(54) **SEALLESS ROTODYNAMIC PUMP**
UNVERSIEGELTE ROTODYNAMISCHE PUMPE
POMPE ROTODYNAMIQUE SANS JOINT D'ETANCHEITE

(43) Date of publication of application: 15.11.1995
(73) Proprietor: THE CLEVELAND CLINIC FOUNDATION, Cleveland, OH 44195 (US)
(72) Inventor: GOLDING, Leonard, A., R., Moreland Hills, OH 44022 (US); SMITH, William, A., Lyndhurst, OH 44124 (US); WADE, Warren, F., Orange Village, OH 44022 (US)
(74) Representative: Kador & Partner
(86) International application number: US9208904
(87) International publication number: WO9409274

(56) References cited:
- WO-A-91/19103
- DE-A- 4 123 433
- FR-A- 2 102 520
- US-A- 3 420 184
- US-A- 3 608 088
- US-A- 3 647 324
- US-A- 3 960 468
- US-A- 4 135 253
- US-A- 4 382 199
- US-A- 4 625 712
- US-A- 4 645 433
- US-A- 4 688 998
- US-A- 4 704 121
- US-A- 4 806 080
- US-A- 4 812 108
- US-A- 5 036 235
- US-A- 5 049 134

## Description

### Background of the Invention

This invention pertains to the art of pumps and more particularly to electrically driven pumps capable for use as heart or blood pumps, or ventricular assist devices.

The invention is applicable to a pump for the pumping of blood of a living person, or animal, to replace or assist the pumping function of the biologic heart. It is capable of being sized and designed to fit inside a pumping chamber of a human heart, e.g. dimensioned on the order of a few inches . However, certain aspects of the invention could be readily configured for use in other environments than a blood pump wherein the presence of a shaft seal or long fluid residence times in the pump would be detrimental to the application.

In the preferred environment, conventional continuous flow rotodynamic blood pumps, which includes centrifugal blood pumps, axial flow blood pumps, or nonpulsatile blood pumps, have suffered from a number of problems. One major difficulty has been the presence of a shaft seal. Loss of blood from the circulatory system, and/or introduction of material of damaging types or quantities into the circulatory system must be prevented. Because of the intended use as life support for a human being, long life and utter reliability are key design goals. Using a contact type seal or fluid purged seal as the shaft seal results in significant difficulties meeting these objectives.

Most commercially available blood pumps, as exemplified by U.S. Pat. Nos. 4,135,253 to Reich, et al., 4,625,712 to Wampler, 3,647,324 to Rafferty, et al., or 4,589,822 to Clausen et al., have a blood pumping impeller mounted on a shaft which penetrates a side wall of the pumping cavity by means of a shaft seal. On the non-blood side of the seal, or drive compartment, is a motor or drive magnet, operating in air or a biocompatible fluid, which rotates the impeller via the shaft. A very common cause of failure of such prior known blood pumps is the eventual leakage of the seal about the shaft which either permits blood to enter into the drive compartment, excessive fluid leakage from the non-blood side into the blood, or both.

Other blood pump structures have been proposed to overcome the problems of blood pump shaft seals. US-A-4,704,121 discloses a noncontacting housing-shaft interface with micron sized clearances that unfortunately introduce significant problems. Attempting to maintain these close dimensional tolerances clearly becomes a manufacturing problem. Additionally, there is a risk of blood leakage if the supply of purge fluid to the non-blood seal of the pump is interrupted.

US-A-5,049,134 discloses a sealless centrifugal blood pump in which a rotatable impeller is supported in a pump housing by fluid bearing during operation.

Rotational movement of the impeller is accomplished with an inverted motor for magnetic driving of the impeller and maintenance of the axial running position of the impeller relative to the housing.

Dorman -4,927,407 suggests using a constant flow pump such as a dynamic perfusion pump to supply a continuous, controlled flow of purge fluid to the seal. The purge fluid flow maintains the seal out of contact with the shaft. Unfortunately, this adds significant complication to the system because of the need for another active control pump and, even then, still requires a purge fluid for normal operation.

Alternatively, it has been suggested to magnetically suspend the impeller in three dimensions. This eliminates the need to breach the pump walls with a shaft to the impeller. US-A-4,704,121 illustrates this approach. However, at best this is a complex system because of its three dimensional nature, and is not presently believed to be commercially feasible.

Still other blood pumps have eliminated a penetrating shaft and accompanying seal by magnetically coupling the pump to a prime mover through a wall of the housing. Dorman - 3,608,088 is an example of one such arrangement from the blood pump field. Although these eliminate leakage, they may amplify other problems also present in shaft sealed designs.

US-A-5,036,235 discloses a brushless DC motor, suitable for a laser scanner or disc drive motor, having a stator core and a rotor which rotate about different centre lines in order to eliminate whirl instability.

For example, high shear stresses around the seal, or around radial and axial bearings, can damage blood if the loads and shear stresses are not very low. Axial magnetic coupling, such as exemplified by Dorman, particularly creates an axial load which must be reacted or countered. Both shear and mechanical friction forces can produce heat that injures susceptible media. With blood, heat produced from the shear and friction forces can also promote deposition, including protein buildup, as well as a conventional clot.

Residence time is also a factor in handling some fluids such as blood. The amount of shear that blood can tolerate is a function of exposure time. Blood coagulation, leading to clots and emboli, also requires a finite time to occur. Thus many pump structures and principles derived from pumps in other fields are not suitable in the blood pump environment since they include relatively stagnant areas where the blood can settle out, react, decompose, or otherwise respond according to the environment and their sensitivity.

Some alternate pump design proposals employ a counter-impeller to prevent backflow along the shaft. This is particularly well known in other pump environments, i.e., non-blood pumps where the dynamic action of a counter-impeller provides a back flow along the shaft. These arrangements hold pumped fluid for long periods in the expeller, thus exposing the pumped fluid to shear. Therefore, these structures are not feasible for use as blood pumps because of all the problems described above associated with long residence times and shear. Further, these arrangements could accelerate leakage into the system if the second impeller discharges its prime of pumped fluid. Even further, these pump arrangements still require additional sealing in order to prevent fluid loss at static or low speed conditions.

The present invention contemplates a new and improved device which overcomes all of the above discussed problems and others to provide a new blood pump.

### Brief Summary of the Invention

There is now provided a new rotodynamic blood pump which is capable of being implanted in a human body to assist or replace the pumping functions of the heart. The pump includes a rotor rotatably received in a pump chamber. The rotor is suspended in the chamber by fluid bearing means, preferably provided by an axial extension of the housing. Means for renewing fluid contact with all wetter surfaces of the chamber is also provided. According to the present invention, this sealless rotodynamic pump comprises a housing (12,18,20) having an inlet (14) and an outlet (16) in fluid communication with a chamber of the housing (18,20), a shaftless rotor (60) received in the chamber for selective rotation relative to the housing (12) without contacting said housing during rotation, drive means for rotating the rotor (60) relative to the housing including a drive element (34,36) and a driven element (62) operatively associated with the rotor (60), and a fluid bearing means defined between the rotor (60) and a housing (12), the rotation of the rotor (60) within the housing (12), causing a flow of fluid through fluid flow passageways (70,74) defined between the housing and the rotor, thereby to continually renew the fluid in contact with all wetted surfaces on the chamber characterised in that the axis (100) of the drive element (34,36) is radially displaced from the axis (102) of the drive housing portion (30) and the axis (102) of the drive housing portion (30) is radially displaced from the axis (103) of the rotor (60), so that a more uniform clearance is maintained between the rotor (60) and the housing (18).

According to a further aspect of the invention, the fluid bearing means includes a groove to increase fluid flow through the bearing means.

According to still another aspect of the present invention, the rotor includes first and second sets of blades. The first blade set has substantially more pumping capacity than the second blade set and defines an impeller or main stage primarily urging the fluid from the inlet to the outlet. The second blade set controls and assists fluid flow through the second passage. Additionally, the second blade set produces a pressure distribution which counteracts that produced by the first blade set, reducing the net force to be reacted in the magnetic coupling.

According to another, yet more limited aspect of the present invention, the rotor includes openings between the primary and secondary flow passages permitting fluid exchange therebetween.

One benefit obtained from the present invention is a sealless pump having a single moving part and improved durability.

Another benefit is the provision of a pump that eliminates flow stasis areas, and maintains surface washing throughout the pump.

A further benefit of the invention is eliminating significant mechanical axial thrust contacts.

Yet another benefit of the invention is a means to stabilize the position and operation of a fluid bearing.

Still other benefits and advantages of the subject invention will become apparent to those skilled in the art upon a reading and understanding of the specification.

### Brief Description of the Drawings

The invention may take physical form in certain parts and arrangements of parts, preferred embodiments of which will be described in detail in this specification and illustrated in the accompanying drawings which form a part hereof and wherein:
FIGURE 1 is a side elevational view of a blood pump formed in accordance with the present invention and particularly showing the pump housing and inlet and outlet passageways;
FIGURE 2 is a left-hand end view taken generally along the lines 2-2 of FIGURE 1;
FIGURE 3 is an enlarged, longitudinal cross-sectional view of a first preferred embodiment of the subject invention;
FIGURE 4 is a cross-sectional view taken generally along lines 4-4 of FIGURE 3;
FIGURE 5 is a cross-sectional view taken generally along lines 5-5 of FIGURE 3;
FIGURE 6 is a schematic illustration of the electromagnetic reaction between a stator and operating rotor in the present invention and is intended to demonstrate the preservation of the desired axial running position of the impeller relative to the housing;
FIGURE 7 is a schematic cross-sectional view taken along line 7-7 of FIGURE 3 particularly illustrating the predetermined radial offset of pump components;
FIGURE 8 is a longitudinal cross-sectional view of an alternate embodiment of the invention; and
FIGURE 9 is another embodiment of the invention showing an alternate version of the drive means.

### Detailed Description of the Preferred Embodiments

Referring now to the drawings wherein the showings are for purposes of illustrating the preferred embodiments of the invention only and not for purposes of limiting same, the FIGURES show a pump **10** comprised of a housing **12**, and having an inlet **14** and an outlet **16**. While the drawings show an axial inlet, and a radial or tangential outlet, these are not essential features of the invention. In the blood application, the pump can be sized for implantation within a living body, and is preferably employed as an assist device for humans. It is to be noted that the pump can be sized so as to even be implantable within a heart chamber, avoiding the substantial problems of larger devices.

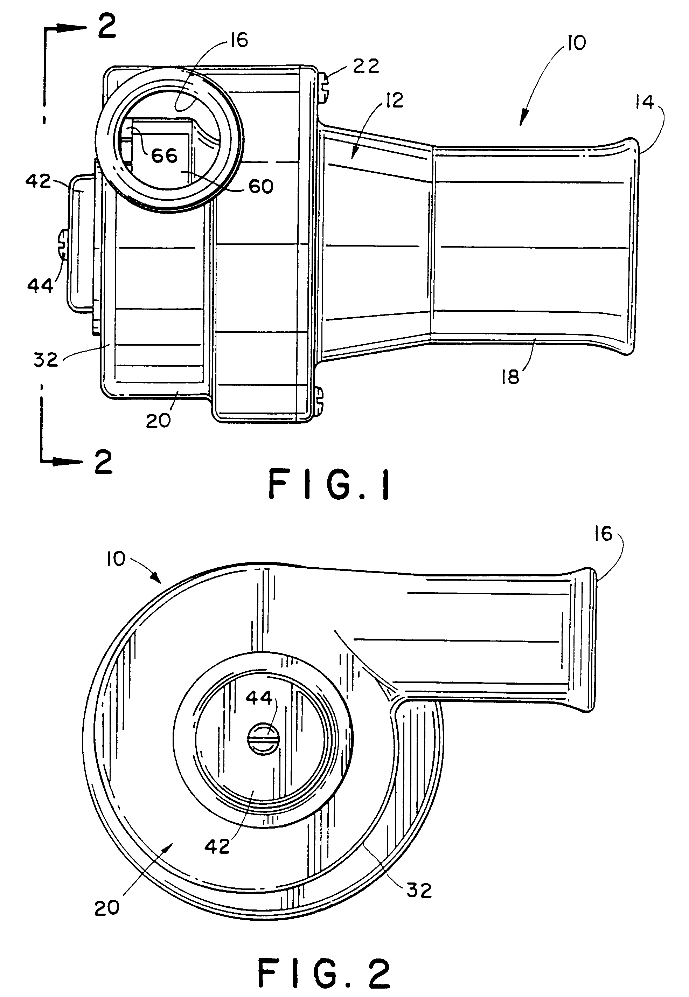

With particular reference to FIGURES 1 - 3, the housing 12 is shown to be more particularly comprised of a rotor portion **18** and a drive housing or outlet portion **20**, which in this particular embodiment partially houses the rotor as well as the drive means. Since in the preferred embodiments the pump is adapted for implantation in the living body, the housing portions are formed from a suitable, biocompatible material such as polished titanium. The housing portions **18, 20** are fastened together with conventional fastening devices **22** and sealed with a conventional sealing device such as an O-ring **24**. The O-ring is positioned in an area of overlapping engagement **26** which has been made an interference fit or bonded so as to be gap free.

The housing portion **20** incorporates an axial extension **30** protruding from end wall **32**. The axial extension receives motor windings **34** and lamination assembly or ferrous stack **36** of an electric motor **38**. The motor is retained in the outlet housing portion by cover **42** and a fastener such as screw **44**. The cover is sealed to the outlet housing portion with O-ring **46**. The extension 30 protrudes a substantial dimension from the end wall, in fact, extending into the rotor housing portion and toward the inlet **14**. This arrangement provides a generally annular pump chamber **48**.

Received over the housing extension **30** is an annular rotor **60**. The rotor includes an encapsulated permanent magnetic assembly **62**, and first and second impeller blade sets **64, 66**. There is no interconnecting shaft between the motor and impeller, i.e. a shaftless rotor. Further, a shaft seal between the motor and the impeller is eliminated, thus obviating many of the problems discussed above in prior art structures. In the preferred embodiment, the permanent magnet assembly **62** in the pump rotor **60** radially couples the rotor to the motor stator (stack and windings) through the non-magnetic wall of housing extension **30**. It should be noted that this arrangement is essentially an inversion of the usual commercial motor arrangement, because the rotating element of the motor, i.e., the permanent magnetic assembly **62**, is larger in diameter and encircles the stationary element, i.e., the stator **34**, **36**. This electric motor serves as the means for driving this embodiment of the invention insofar as it serves to create rotational motion of the pump rotor relative to the housing. The stator assembly is the drive element and the permanent magnet assembly the driven element of this version of a drive means.

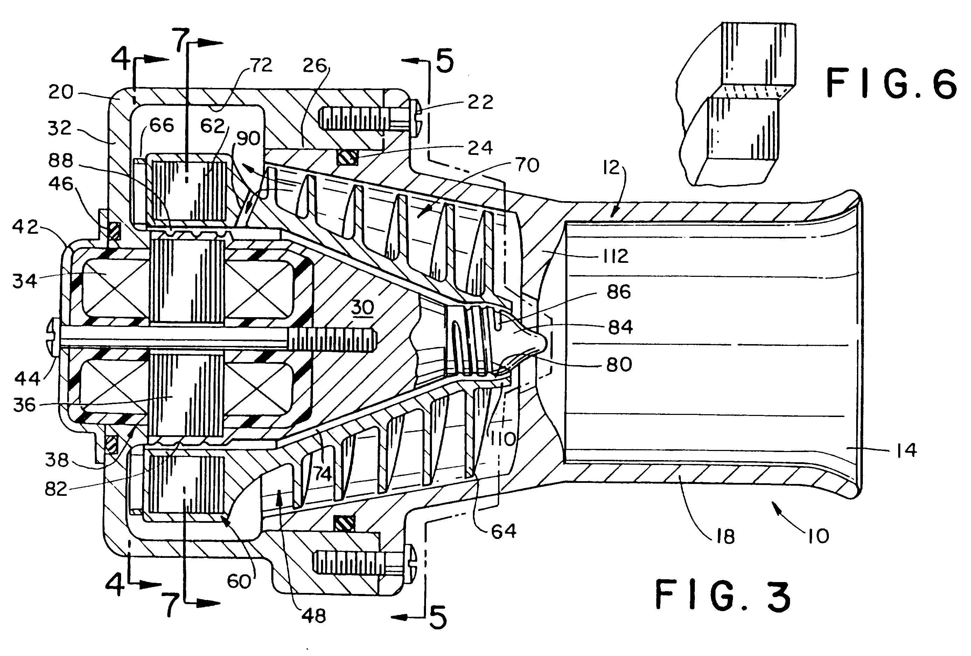

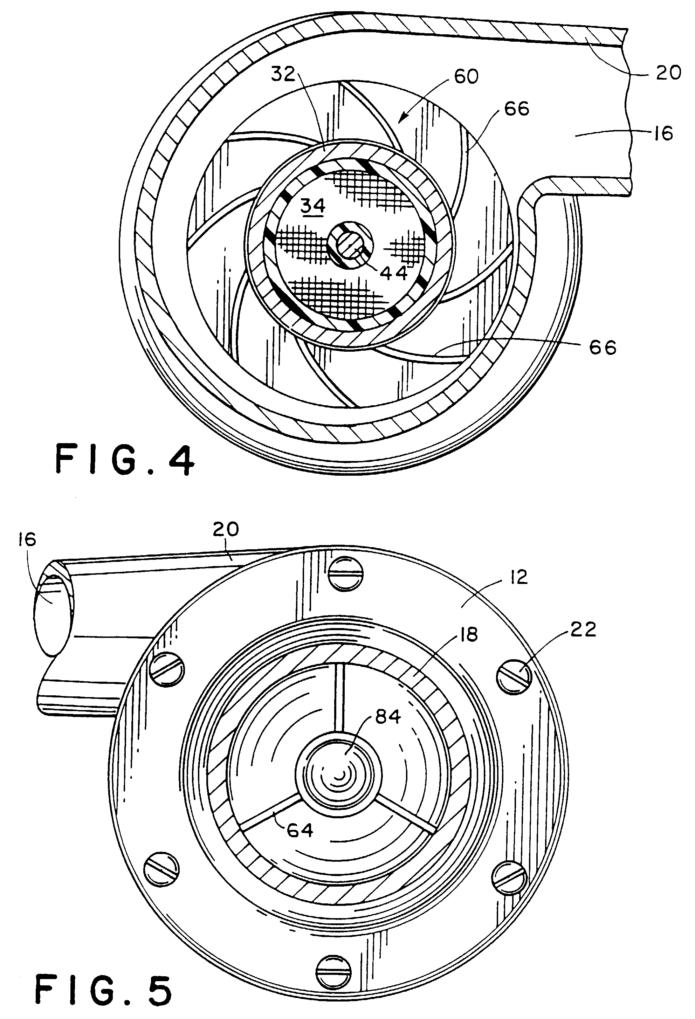

With particular reference to FIGURES 3 and 4, the first or primary impeller blade set **64** includes a plurality of mixed flow impeller blades. Radial flow or axial flow blade arrangements could also be encompassed within the scope of the invention. The impeller shown is a three-bladed variable lead screw. The secondary impeller blade set **66** is comprised of a plurality of radial flow impeller blades in this design embodiment.

The placement of rotor **60** in the housing **12** defines a continuous, first fluid passage **70** between the rotor **60** and the interior wall of the housing, which traverses from the inlet **14** to the annular output collector **72** of the pump chamber. A continuous second passage fluid passage **74** is formed between the housing extension **30** and the inside diameter of pump rotor **60**. The second passage **74** is has a generally large clearance, perhaps 0.508-0.762 mm (0.020 - 0.030 inch), compared to the flow to be passed, but narrows to approximately 0.0762-0.127mm (0.003 - 0.005 inch) at opposite ends of the rotor to define first and second fluid bearings **80, 82** during operation of the pump. The first bearing 80 is located at the terminal end **84** of the portion of the motor housing extension **30** facing the inlet **14**. This terminal end portion **84** has a generally cone-like configuration and includes one or more extended helical grooves **86** to increase the lubricating and cooling flow through the fluid bearing **80**. The second fluid bearing **82** similarly has a helically configured grooving **88** on the outer wall of the motor housing extension **30** to increase the bearing wash flow.

For improved pumping that avoids fluid damage or deposition due to sluggish or non-existing flow velocities through second passage **74**, a continuous washing flow is required. The second set of impeller blades **66** scavenges blood from the second passage, discharging it to the collector **72**. To prevent excessive pressure drop, a plurality of circumferentially spaced openings **90** extend generally radially between the first and second fluid passages to permit fluid to flow from the first to the second passage. Under the action of the pressure rise produced by the first blade set **64**, flow traverses from openings **90** to impeller inlet **14**, along passage **74**. The second blade set also draws fluid from openings **90**, through bearing **82**, past end wall **32** and discharges the fluid into the collector **72**.

With particular reference to FIGURE 4, it may be seen that the annular collector chamber **72** can be spirally offset relative to the generally circular dimension of the rotor **60**. Such a configuration is generally conventional for the volume of a rotodynamic pump, but the present invention may also be used with other forms of discharge collector.

Because the annular pump rotor **60** is freely received in the housing **12**, it is important that its motion be controlled so that damage to the pumped fluid or the mechanical components not occur in close clearance areas, such as bearings **80, 82**, or at interior walls of the housing. The symmetrical design of the pump permits the radial load to be low, which results in a significant fluid film thickness on the order of 0.0254 mm (0.001 inch) at bearings **80, 82**. This avoids mechanical wear on the pump components, and minimizes fluid shear of the blood, both of which are obviously detrimental to the intended use of the pump. On the other hand, if the load is too iow, the bearings can go into a well-known whirl mode, destroying the film thickness and the bearings. In this operating mode, instead of rotating around a fixed axis, the rotor rolls 360 degrees around the stator, wearing all surfaces of the rotor and stator.

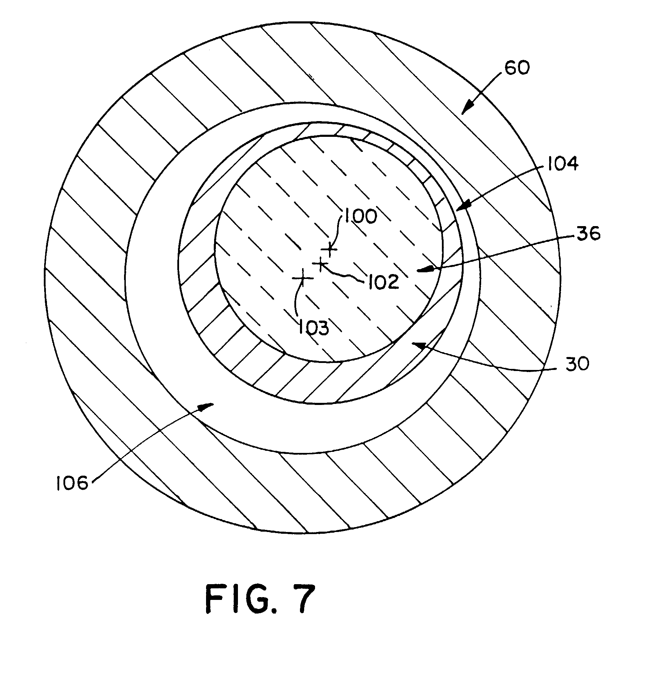

The subject invention overcomes this problem by purposely and deliberately radially offsetting the centerline **100** of the motor stator relative to the centerline **102** of the drive housing portion **30** (FIGURE 7). As a result of this offset, magnetic forces are higher at region **104**, and lower at region **106**, resulting in a known, controlled magnitude and direction of bearing loading. For example, preliminary tests of pump prototypes in accordance with the subject invention provide an offset on the order of 0.0254-0.0508 mm (0.001 - 0.002 inch) which results in magnetic forces of a few tenths of a pound at **104** and at **106**. Because of this known force magnitude and direction, it is possible to calculate the running position of the rotor **60** (as represented by numeral **103**, which is also representative of the centerline of the drive housing portion **30**) and establish another centerline shift between the drive housing portion **30** and the rotor housing portion **18**, such that the clearance between the impeller blades **64** and the housing wall is held more uniform around the circumference.

The axial motion of the pump rotor **60** must also be controlled to maintain a desired position of the rotor. The summation of pressure forces acting along the first and second fluid passages **70, 74**, respectively, will tend to move the rotor relative to end wall **32**. The magnetic attraction forces between the stator **34** and magnet assembly **38** are designed to be sufficient to overcome this tendency, even with slight variation of movement. FIGURE 6 illustrates this operation. Axial motion is further limited during transients by the interaction of conical extension **110** on the rotor **40** with the housing cone **84** and inlet stop means which resemble stator blades **112.** Should motions occur outside of normal limits, the rotor will contact stop means at controlled points of small diameter and consequent low rubbing velocity. Alternatively, stop means 112 could be replaced by reconfiguring the interior wall at a slightly different angle than that on rotor **60**, such that any contact would be in a local, small diameter area adjacent to inlet **14**.

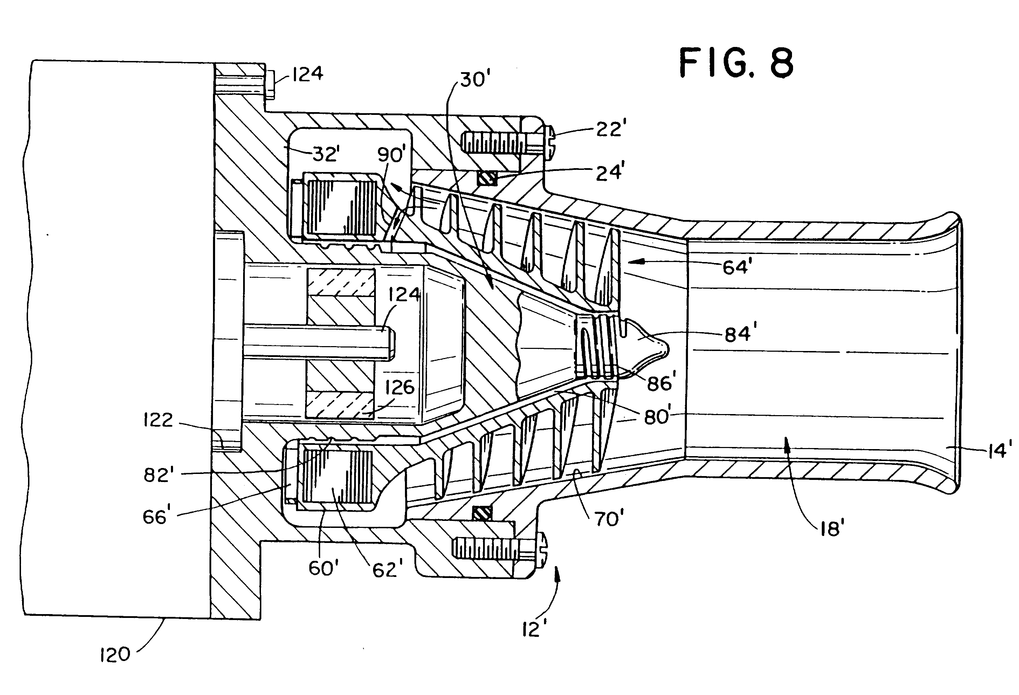

FIGURE 8 contains an alternative blood pump in accordance with the teachings of the subject invention. Like elements are referred to by like numerals with a primed (') suffix, while new elements are referenced by new numerals. Pump housing **12'** is guidedly mounted to an external prime mover **120** by pilot diameter **122** and quick connect fasteners **124**. The prime mover could be an electric motor, or other suitable means of converting energy to rotary motion of the shaft **124**. Fixedly mounted to the shaft **124** of the prime mover is a driver magnet assembly **126**. This forms a magnetic coupling with driven magnet assembly **62'** as will be understood by those skilled in the art. The driver and driven magnets rotate synchronously under the urging of the motor **120**. The motor and magnet assembly functionally replace the stator **34** and coils **36** previously discussed as fixedly mounted in the housing **20** and together constitute the drive element of the drive means of this embodiment of the invention. As other details of construction of this embodiment are substantially identical to those already described except for the omission of the conical extension 110 and stop means **112**, they will not be repeated here.

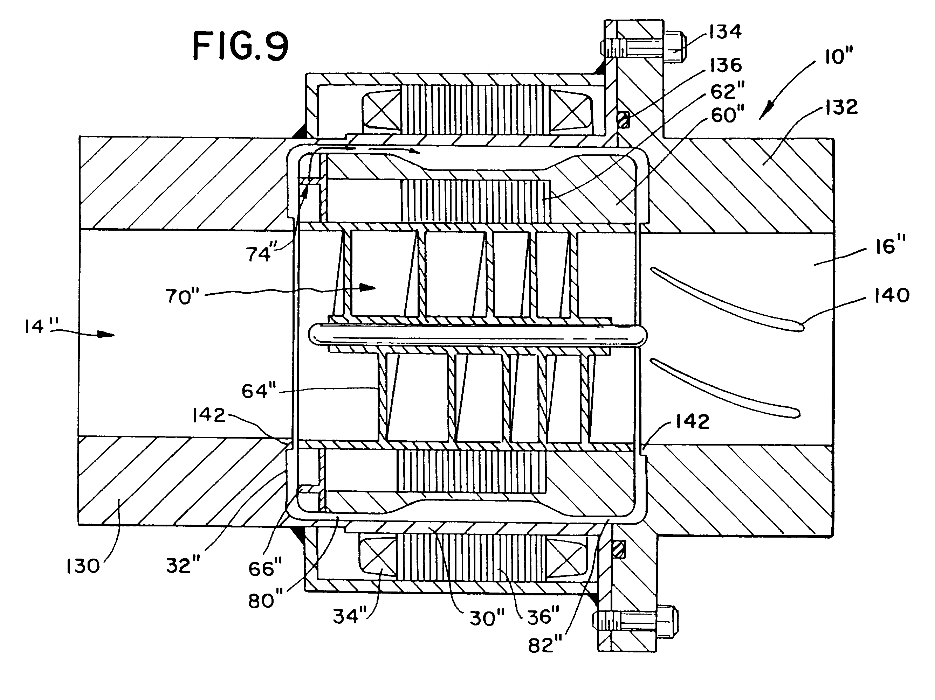

FIGURE 9 illustrates an inverted embodiment of the invention, apparently different in configuration but generally working according to the same principles described above. Like numerals with a double primed suffix (") are used to identify like elements while new numerals refer to new elements.

The pump has a housing **10"** having an inlet **14"** and an outlet **16"**. The housing particularly comprises a drive housing portion **130** and a discharge housing portion **132**. The housings are retained together by conventional fasteners **134** and sealed by O-rings **136**. Installed within the housing portion **130** is a motor stator **36"** and associated windings **34"**. Received in the housing portion is pump rotor **60"**, which comprises permanent magnet assembly **62"**, and impeller blade sets **64", 66"**. The primary blade set **64"**, though, is of axial flow geometry. It is further noted that the motor is now of conventional arrangement with the stator being of larger diameter and surrounding the magnet assembly **62"**. Axial extension **30"** of the end wall **32"** of housing forms radial bearing means **80", 82"** to support the rotor.

As received in the housing **10"**, the pump rotor defines primary and secondary flow paths **70", 74"**, respectively. The primary impeller blades **64"** are located in the first flow passage, and urge blood from inlet **14"** to outlet **16"**. Stator blades **140** in the outlet housing slow the blood from and convert velocity into pressure energy. Secondary pump blades **66"** urge blood from the inlet through passage **74"** to the outlet. In a variation of this design, the secondary blades could be deleted and the wall separating the primary and secondary flow passages eliminated. In this case the primary impeller blades would urge flow through both the primary and "secondary" passage, and the bearing surfaces would be formed by the blade tips. Passage **74"** is narrowed at each end of the pump rotor to form first and second fluid bearings **80", 82"**. As in the previous discussion of FIGURE 7, centerline shift can be used to provide a stable load for these bearings. The net force resulting from the summation of pressures acting on the pump rotor is primarily reacted by the magnetic attraction of the stator and magnet assembly as diagrammed in FIGURE 6. Transient axial motions, in turn, can be limited by a plurality of struts **142** on the housing.

The invention has been described with reference to the preferred embodiments. Obviously, modification and alternations will occur to others upon the reading and understanding of the specification. It is our intention to include all such modifications and alterations insofar as they come within the scope of the appended claims or the equivalents thereof.

## Claims

1. A sealless rotodynamic pump for pumping the blood of a living person or animal to assist or replace the pumping action of the heart, comprising a housing (12,18,20) having an inlet (14) and an outlet (16) in fluid communication with a chamber of the housing (18,20) a shaftless rotor (60) received in the chamber for a selective rotation relative to the housing (12) without contacting said housing during rotation, drive means for rotating the rotor (60) relative to the housing including a drive element (34,36) and a driven element (62) operatively associated with the rotor (60), and a fluid bearing means defined between the rotor (60) and a housing (12), the rotation of the rotor (6) within the housing (12) causing a flow of fluid through fluid flow passageways (70,74) defined between the housing and the rotor, thereby to continually renew the fluid in contact with all wetted surfaces on the chamber characterised in that the axis (100) of the drive element (34,36) is radially displaced from the axis (102) of the drive housing portion (30), and the axis (102) of the drive housing portion is radially displaced from the axis (103) of the rotor (60) so that a more uniform clearance is maintained between the rotor (60) and the housing (18).

2. The pump as defined in claim 1 further comprising an opening (90) through the rotor connecting the first and second passages for permitting flow therebetween.

3. The blood pump as defined in claim 1 wherein the rotor includes first (64) and second (66) sets of blades, the first blade set disposed in the first passage (70) to pump fluid from the inlet to the outlet.

4. The pump as defined in claim 3 wherein the second blade set (66) scavenges fluid from the second passage (74) to enhance fluid through flow.

5. The blood pump as defined in claim 1 wherein the second passage (74) is narrowed at one end to define a fluid bearing (82).

6. The pump as defined in claim 5, further comprising a groove (88) in a side wall of the second passage to increase the area for flow through the fluid bearing.

7. The pump as defined in claim 5 wherein the second passage is narrowed at the other end to define a second fluid bearing (80).

8. The pump as defined in claim 7 further comprising a groove (86) in a side wall of the second passage to increase the area for flow through the fluid bearing.

9. The pump as defined in claim 1 wherein the drive means includes an electric motor stator (36) and winding (34) in the housing portion and a surrounding permanent magnet assembly (62) in the rotor.

10. The pump as defined in claim 1 wherein the magnetic attraction of the drive and driven elements of the rotating means define a thrust reaction means to resist longitudinal displacements caused by axial fluid, gravitational inertial or other forces.

11. The pump as defined in claim 1 further comprising means for limiting axial travel of the rotor relative to the housing.

12. The pump as defined in claim 11 wherein the limiting means includes a contact surface (112) adapted to selectively contact the rotor.

13. The pump as defined in claim 1 wherein the drive means comprises a magnetic coupling in combination with a prime mover (120).

14. The pump as defined in any preceding claim wherein one end (110) of the rotor is conically narrowed and cooperates with strut means (112) on the housing for limiting axial travel of the rotor relative to the housing.

## Patentansprüche

1. Dichtungslose rotodynamische Pumpe zum Pumpen des Bluts einer lebenden Person oder eines Tiers, um die Pumpwirkung des Herzens zu unterstützen oder zu ersetzen, mit einem Gehäuse (12, 18, 20), das einen Einlaß (14) und einen Auslaß (16) besitzt, die mit einer Kammer des Gehäuses (18, 20) in einer Fluidverbindung stehen, einem wellenlosen Rotor (60), der in der Kammer aufgenommen ist, um sich relativ zum Gehäuse (12) wahlweise zu drehen, ohne das Gehäuse während der Drehung zu berühren, einer Antriebseinrichtung zum Drehen des Rotors (60) relativ zum Gehäuse, die ein Antriebselement (34, 36) und ein angetriebenes Element (62), das dem Rotor (60) funktional zugeordnet ist, enthält, und einer Fluidlagereinrichtung, die zwischen dem Rotor (60) und dem Gehäuse (12) definiert ist, wobei die Drehung des Rotors (60) im Gehäuse (12) eine Fluidströmung durch zwischen dem Gehäuse und dem Rotor definierte Fluidströmungsdurchlässe (70, 74) hervorruft, um dadurch das Fluid, das mit sämtlichen benetzten Oberflächen der Kammer in Kontakt ist, ununterbrochen zu erneuern, dadurch gekennzeichnet, daß die Achse (100) des Antriebselements (34, 36) in bezug auf die Achse (102) des Antriebsgehäuseabschnitts (30) radial versetzt ist und die Achse (102) des Antriebsgehäuseabschnitts in bezug auf die Achse (103) des Rotors (60) radial versetzt ist, so daß zwischen dem Rotor (60) und dem Gehäuse (18) ein gleichmäßigerer Zwischenraum aufrechterhalten wird.

2. Pumpe nach Anspruch 1, ferner mit einer Öffnung (90) durch den Rotor, die den ersten und den zweiten Durchlaß miteinander verbindet, um zwischen ihnen eine Strömung zuzulassen.

3. Blutpumpe nach Anspruch 1, wobei der Rotor eine erste (64) und eine zweite (66) Gruppe von Schaufeln enthält, wobei die erste Schaufelgruppe im ersten Durchlaß (10) angeordnet ist, um Fluid vom Einlaß zum Auslaß zu pumpen.

4. Pumpe nach Anspruch 3, wobei die zweite Schaufelgruppe (66) Fluid vom zweiten Durchlaß (74) spült, um den Fluiddurchfluß zu steigern.

5. Blutpumpe nach Anspruch 1, wobei der zweite Durchlaß (74) an einem Ende verengt ist, um ein Fluidlager (82) zu definieren.

6. Pumpe nach Anspruch 5, ferner mit einer Nut (88) in einer Seitenwand des zweiten Durchlasses, um die Fläche der Strömung durch das Fluidlager zu erhöhen.

7. Pumpe nach Anspruch 5, wobei der zweite Durchlaß am anderen Ende verengt ist, um ein zweites Fluidlager (80) zu definieren.

8. Pumpe nach Anspruch 7, ferner mit einer Nut (86) in einer Seitenwand des zweiten Durchlasses, um die Fläche der Strömung durch das Fluidlager zu erhöhen.

9. Pumpe nach Anspruch 1, wobei die Antriebseinrichtung einen Elektromotor-Stator (36) und eine Elektromotor-Wicklung (34) im Gehäuseabschnitt sowie eine umgebende Permanentmagnetbaueinheit (62) im Rotor enthält.

10. Pumpe nach Anspruch 1, wobei die magnetische Anziehung des Antriebselements und des angetriebenen Elements der Rotationseinrichtung eine Schubreaktionseinrichtung definiert, um longitudinalen Verschiebungen zu widerstehen, die durch axiale Fluidkräfte, die Schwerkraft, Trägheitskräfte oder andere Kräfte bewirkt werden.

11. Pumpe nach Anspruch 1, ferner mit einer Einrichtung zum Begrenzen der axialen Bewegung des Rotors relativ zum Gehäuse.

12. Pumpe nach Anspruch 11, wobei die Begrenzungseinrichtung eine Kontaktoberfläche (112) enthält, die so beschaffen ist, daß sie den Rotor wahlweise berührt.

13. Pumpe nach Anspruch 1, wobei die Antriebseinrichtung eine magnetische Kopplung in Kombination mit einer Antriebsmaschine (120) enthält.

14. Pumpe nach irgendeinem vorangehenden Anspruch, wobei sich ein Ende (110) des Rotors konisch verjüngt und mit einer Strebeneinrichtung (112) am Gehäuse zusammenwirkt, um die axiale Bewegung des Rotors relativ zum Gehäuse zu begrenzen.

## Revendications

1. Pompe rotodynamique sans joint d'étanchéité pour pomper le sang d'une personne vivante ou d'un animal vivant afin d'assister ou remplacer l'action de pompage du coeur, comprenant un boîtier (12, 18, 20) comportant une entrée (14) et une sortie (16) en communication de fluide avec une chambre du boîtier (18, 20), un rotor sans arbre (60) reçu dans la chambre pour une rotation sélective par rapport au boîtier (12) sans contact avec le boîtier pendant la rotation, des moyens d'entraînement pour faire tourner le rotor (60) par rapport au boîtier comprenant un élément entraînant (34, 36) et un élément entraîné (62) associés de façon opérationnelle avec le rotor (60), et des moyens formant paliers de fluide définis entre le rotor (60) et le boîtier (12), la rotation du rotor (6) dans le boîtier (12) provoquant un débit de fluide à travers des passages (70, 74) définis entre le boîtier et le rotor, de manière à renouveler continuellement le fluide en contact avec toutes les surfaces mouillées de la chambre, caractérisée en ce que l'axe (100) de l'élément entraînant (34, 36) est déplacé radialement par rapport à l'axe (102) de la partie de boîtier d'entraînement (30), et l'axe (102) de la partie de boîtier d'entraînement est déplacée radialement par rapport à l'axe (103) du rotor (60) si bien qu'un jeu plus uniforme est maintenu entre le rotor (60) et le boîtier (18).

2. Pompe selon la revendication 1, comprenant de plus une ouverture (90) ménagée à travers le rotor raccordant les premier et second passages pour permettre le passage d'un débit entre eux.

3. Pompe à sang selon la revendication 1, dans laquelle le rotor comprend des premier (64) et second (66) jeux d'ailettes, le premier jeu d'ailettes étant disposé dans le premier passage (70) pour pomper du fluide à partir de l'entrée et jusqu'à la sortie.

4. Pompe selon la revendication 3, dans laquelle le second jeu d'ailettes (66) aspire du fluide à partir du second passage (74) pour augmenter le débit de fluide.

5. Pompe à sang selon la revendication 1, dans laquelle le second passage (74) est rétréci à une extrémité pour délimiter un palier de fluide (82).

6. Pompe selon la revendication 5, comprenant de plus une rainure (88) ménagée dans une paroi latérale du second passage pour augmenter la zone de passage de débit à travers le palier de fluide.

7. Pompe selon la revendication 5, dans laquelle le second passage est rétréci à l'autre extrémité pour délimiter un second palier de fluide (80).

8. Pompe selon la revendication 7, comprenant de plus une rainure (86) ménagée dans une paroi latérale du second passage pour augmenter la zone de passage de débit à travers le palier de fluide.

9. Pompe selon la revendication 1, dans laquelle les moyens d'entraînement comprennent un stator (36) et un enroulement (34) de moteur électrique agencé dans la partie de boîtier et un ensemble entourant à aimant permanent (62) agencé dans le rotor.

10. Pompe selon la revendication 1, dans laquelle l'attraction magnétique des éléments entraînant et entraîné des moyens de rotation définit des moyens de réaction de poussée pour résister aux déplacements longitudinaux causés par des forces axiales, inertielles, gravitationnelles, de fluide ou autres.

11. Pompe selon la revendication 1, comprenant de plus des moyens pour limiter le trajet axial du rotor par rapport au boîtier.

12. Pompe selon la revendication 11, dans laquelle les moyens de limitation comprennent une surface de contact (112) adaptée pour être en contact sélectivement avec le rotor.

13. Pompe selon la revendication 1, dans laquelle les moyens d'entraînement comprennent un couplage magnétique en combinaison avec une machine motrice (120).

14. Pompe selon l'une quelconque des revendications précédentes, dans laquelle une extrémité (110) du rotor est rétrécie de façon conique et coopère avec des moyens d'entretoisement (112) dans le boîtier pour limiter le trajet axial du rotor par rapport au boîtier.
